(19) Europäisches Patentamt
European Patent Office
Office européen des brevets

(11) Veröffentlichungsnummer : **0 003 229**
**B1**

(12) **EUROPÄISCHE PATENTSCHRIFT**

(45) Veröffentlichungstag der Patentschrift : **02.12.81**

(51) Int. Cl.³ : **C 07 H 19/04**// C07D405/04

(21) Anmeldenummer : **78101710.8**

(22) Anmeldetag : **15.12.78**

(54) **Neues Verfahren zur Herstellung von Nucleosiden.**

(30) Priorität : **20.12.77 DE 2757365**

(43) Veröffentlichungstag der Anmeldung : **08.08.79 (Patentblatt 79/16)**

(45) Bekanntmachung des Hinweises auf die Patenterteilung : **02.12.81 Patentblatt 81/48**

(84) Benannte Vertragsstaaten : **BE CH DE FR GB IT NL SE**

(56) Entgegenhaltungen :
**DE - A - 2 508 312**
**JUSTUS LIEBIGS ANNALEN DER CHEMIE**
**Verlag Chemie Weinheim, 1976,**
**Heft 1, Seiten 745-761**

(73) Patentinhaber : **SCHERING AKTIENGESELLSCHAFT**
**Berlin und Bergkamen**
**Müllerstrasse 170/178 Postfach 65 0311**
**D-1000 Berlin 65 (DE)**

(72) Erfinder : **Vorbrüggen, Helmut, Prof. Dr.**
**Wilkestrasse 7**
**D-1000 Berlin 27 (DE)**

**EP 0 003 229 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Neues Verfahren zur Herstellung von Nucleosiden

Beschreibung

Bei den bekannten Nucleosidsynthesen nach der Silylmethode, wie sie z.B. im DBP 1 919 307 oder in der DOS 2 508 312 beschrieben werden, war es unumgänglich, die Nucleosidbasen, insbesondere die Pyrimidin-Basen Uracil, 2-Thiouracil, Cytosin usw. sowie die Purinbase Adenin, $N^6$-Benzoyladenin, Hypoxanthin, Xanthin und Guanin vor der eigentlichen Nucleosidsynthese zu silylieren und die entstandenen feuchtigkeitsempfindlichen persilylierten Nucleosidbasen erst in einem zweiten Reaktionsschritt mit geschützten 1-Halogenzuckern, 1-O-Acylzuckern und insbesondere 1-O-Acylzuckern in Gegenwart von Friedel-Crafts-Katalysatoren wie $SnCl_4$, $TiCl_4$, $ZnCl_2$, $BF_3$-Ätherat, $AlCl_3$, $SbCl_5$ oder Trimethylsilylperfluoralkan sulfonaten bzw. $(CH_3)_3SiClO_4$ umzusetzen.

Aus Liebigs Annalen der Chemie *1976,* 745 ist eine einstufige Silylierung bekannt. Hierbei handelt es sich jedoch um ein Verfahren, das im Gegensatz zu dem in der DE-OS 25 083 10 beschriebenen Verfahren die Silyloxygruppe als « leaving group » einführt, weil die OH-Gruppe nicht mit Aminen reagiert.

Ausser in der DE-OS 25 08 310 werden auch in US 3 354 160, US 3 352 849, US 3 531 464, US 3 817 980, US 3 748 320, US 4 082 911, US 3 708 469, in Z. Chem. (1964), 303 und in Chem. Ber. (1968), 1 095 stets nur 2-stufige Nucleosidverfahren beschrieben, in denen die OH-, SH-, und $NH_2$-Gruppen von der Reaktion mit Zuckern geschützt werden und die für Zucker reaktiven Stellen am Molekül unangetastet bleiben. In diesen 10 genannten Publikationen mit weit über 100 Beispielen sind keine Hinweise auf Einstufenverfahren für die Herstellung an Nucleosiden zu finden.

Es wurde nun gefunden, daß sich beide Reaktionsschritte, d.h. Persilylierung der Nucleosidbasen und darauf folgende Nucleosidsynthese mit den geschützten Zuckerderivaten in Gegenwart von Friedel-Crafts-Katalysatoren bei einer bestimmten Dosierung des Silylierungsreagenz überraschenderweise in einem Schritt zusammenfassen lassen und daß sich die bisher übliche Silylierung der Nucleosidbase wie auch die Silylierung der Perfluoralkansulfonsäuren, der Perchlorsäure und der Borfluorwasserstoffsäure bzw. der Salze dieser Säuren zu den aus der DOS 2 508 312 bekannten Friedel-Crafts-Katalysatoren ebenfalls erübrigt.

Die Erfindung betrifft somit ein Verfahren zur Herstellung von Nucleosiden durch Umsetzung der entsprechenden Nucleosidbasen mit einem 1-O-Acyl-, 1-O-Alkyl- oder 1-Halogenderivat eines geschützten monomeren oder oligomeren Zuckers in Gegenwart eines Katalysators, dadurch gekennzeichnet, daß man die Silylierung der Base und die Umsetzung mit dem Zuckerderivat in einem Schritt durchführt, wobei man zur Silylierung der Base pro Hydroxy-, Mercapto- oder Aminogruppe ein Gemisch von etwa 0,66 Äquivalenten Hewamethyldisilazan (HMDS) und etwa 0,33 Äquivalenten Trimethylhalogensilan (mit Chlor und Brom als Halogen), bevorzugt mit Trimethylchlorsilan, verwendet. Als Katalysatoren verwendet man typische Friedel-Crafts-Katalysatoren wie $SnCl_4$, $TiCl_4$, $ZnCl_2$, $BF_3$-Ätherat, $AlCl_3$, $SbCl_5$ sowie Trimethylsilylperchlorat $[(CH_3)_3SiClO_4]$, $(CH_3)_3SiSO_3C_nF_{2n+1}$ mit $n = 1-10$, bevorzugt $n = 1-4$, bzw. $(CH_3)_3SiBF_4$ (nach Literaturangaben von Evers in J. inorg. Chem. *13,* 239 (1960) instabil), die man *in situ* aus den freien Säuren bzw. ihren Salzen, insbesondere den Alkali-, wobei Natrium und Kalium bevorzugt sind, oder Ammoniumsalzen, mit Hilfe des im Überschuß zugesetzten Silylierungsmittels, insbesondere Trimethylchlorsilan (TCS), herstellt.

Im Sinne des erfindungsgemäßen Verfahrens sind alle organischen Basen, wie sie dem Fachmann allgemein bekannt sind, anwendbar.

Beispielsweise geeignet sind organische Basen der Formeln

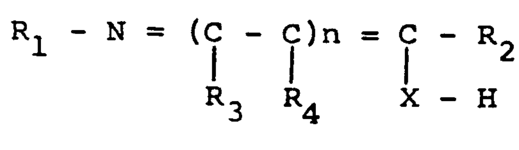

(Ia)

oder

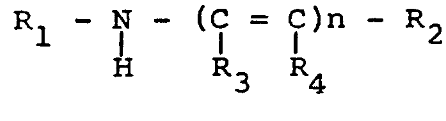

(Ib)

worin X die Atome O oder S und n die Zahl 0 oder 1 und $R_1$ und $R_2$ jeder für sich einen beliebigen gesättigten oder ungesättigten, gegebenenfalls substituierten organischen Rest oder gemeinsam einen zweiwertigen organischen Rest, der ein oder zwei Stickstoffatome enthalten kann, und $R_3$ und $R_4$ jeder für sich Wasserstoff, einen Alkyl-, Alkoxycarbonyl-, Alkylaminocarbonylrest oder gemeinsam die Reste

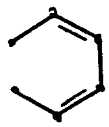, 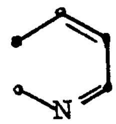, 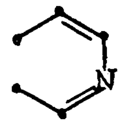, 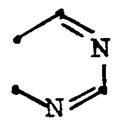, 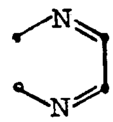, 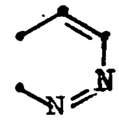,

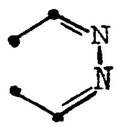, 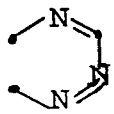, 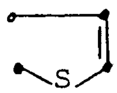, 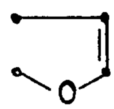, 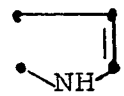, 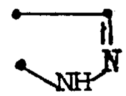

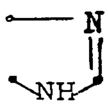 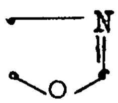 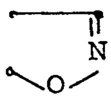 oder 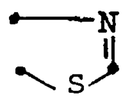 die in üblicher Weise substituiert sein können, bedeuten.

Bedeutet $R_1$ und $R_2$ einen beliebigen organischen Rest, handelt es sich insbesondere um niedere Alkylgruppen, vorzugsweise mit 1-4 Kohlenstoffatomen. Beispielsweise genannt seien der Methyl-, Äthyl-, Propyl- oder Butylrest sowie Aryl- oder Aralkylgruppen.

Die zweiwertigen Reste $R_1$, $R_2$ sowie $R_3$ und $R_4$ können beispielsweise folgende Substituenten enthalten :

niedere Alkyl-, Trifluormethyl-, Acyl-, Hydroxy-, Alkoxy-, Acyloxy-, Carboxyl-, Carboxamido-, Alkoxycarbonyl-, Dialkylaminocarbonyl-, Amino-, Nitrogruppen, Nitriloxogruppen oder Halogenatome.

Bevorzugte Ausgangsprodukte sind organische Basen, in denen $R_1$ und $R_2$ ringverknüpft sind und insbesondere in der Weise, dass die heterocyclische Base fünf oder sechs Atome, davon ein bis drei Stickstoffatome, im Ring enthält.

Die organischen Basen gemäß der Formeln Ia und Ib leiten sich somit bevorzugt von folgenden heterocyclischen Basen ab :

Uracil, Cytosin, 6-Azauracil, 2-Thio-6-azauracil, Thymin, N-Acyl-Adenin, Guanin, Lumazin, Imidazol, Pyrazin, Thiazol, Triazol, die gegebenenfalls durch einen oder mehrere der oben genannten Reste $R_1$, $R_2$ sowie $R_3$ und $R_4$ substituiert sein können.

Für den Fall, in dem $R_1$ und $R_2$ miteinander ringverknüpft sind, bedeutet der zweiwertige Rest $R_1$ $R_2$ insbesondere

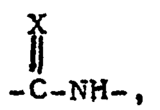 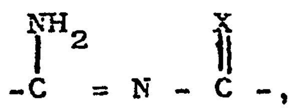 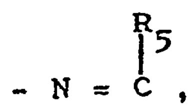 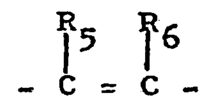

$-CH=N-$ , 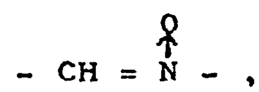 , $-O-CO-$

(wenn n = 1) oder $-NH-CO-CH=N-$, 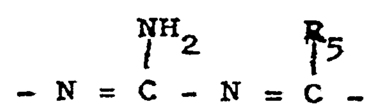

oder 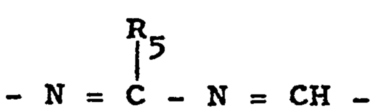

(wenn n = 0), wobei X die oben angegebene Bedeutung hat und $R_5$ und $R_6$ Wasserstoff, einen Alkyl-, Alkoxycarbonyl- oder Alkylaminocarbonylrest bedeuten.

Die verfahrensgemäss eingesetzten Zuckerderivate leiten sich vorzugsweise ab von Ribose, Desoxyribose, Arabinose und Glucose bzw. von 2-substituierten Derivaten des Tetrahydrofurans oder Tetrahydropyrans. Außerdem sollen auch peracylierte Oligomere von Zuckern, insbesondere der Glucose, wie z.B. peracetylierte Cellobiose, Cellotriose bzw. Polymere mit 10-50 Glucoseeinheiten eingesetzt werden.

Zweckmässigerweise werden alle freien Hydroxygruppen der Zucker geschützt. Als Zuckerschutzgruppen eignen sich die in der Zuckerchemie geläufigen Schutzgruppen, wie zum Beispiel die Acyl-, Benzoyl-, p-Chlorbenzoyl-, p-Nitrobenzoyl-, p-Toluyl-, und Benzylgruppen.

In den verfahrensgemäss erhaltenen Nucleosiden ist der freie oder geschützte Zuckerrest mit dem Stickstoffatom vorzugsweise β-glycosidisch verknüpft.

Sollen verfahrensgemäss Nucleoside hergestellt werden, die O-Acylgeschützte Zuckerreste enthalten, so kommen ausser den bereits genannten Schutzgruppen noch unter anderem die Reste folgender Säuren in Betracht: Propionsäure, Buttersäure, Valeriansäure, Capronsäure, Önenthsäure, Undecylsäure, Ölsäure, Pivalinsäure, Cyclopentylpropionsäure, Phenylessigsäure, Adamantancarbonsäure.

Das erfindungsgemässe Verfahren ist allgemein anwendbar zur Herstellung von Nucleosiden. Bevorzugte Verfahrensprodukte sind Nucleoside der allgemeinen Formel II

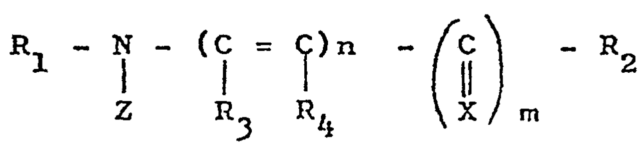

worin $R_1$, $R_2$, $R_3$, $R_4$, X und n die oben angegebene Bedeutung besitzen und Z einen freien oder geschützten Zuckerrest und m die Zahl 0 oder 1 bedeuten. Die weiter unten angegebenen Reaktionsbedingungen sind beispielhafte Ausführungsformen der Erfindung. Die verfahrensgemäss herstellbaren Nucleoside und insbesondere die Verfahrensprodukte der Formel II, sind biologisch aktiv. Infolge der spezifischen Löslichkeit können sie je nach Wahl des Substituenten entweder systemisch als wässrige oder alkoholische Lösung gegeben werden, oder lokal als Salbe oder Gelee angewendet werden.

Die Verbindungen haben — je nach Ausgangsverbindung — zum Beispiel enzymhemmende, antibakterielle, antivirale, cytostatische, antipsoriatische, entzündungshemmende Wirkung.

Die Umsetzung der organischen Basen, z.B. der Basen der Formel Ia oder Ib, mit einem 1-O-Acyl-, 1-O-Alkyl- oder 1-Halogen-Derivat eines geschützten Zuckers in Gegenwart des Silylierungsmittels und Friedel-Crafts-Katalysators bzw. der für eine *in situ* Bildung dieses Katalysators notwendigen Chemikalien erfolgt in einem organischen, indifferenten Lösungsmittel, beispielsweise in Äthylen, Äthylenchlorid, Chloroform, Acetonitril, Benzol, Toluol, Dioxan, Tetrahydrofuran, Dimethylformamid, Schwefelkohlenstoff, Chlorbenzol, Sulfolan oder geschmolzenem Dimethylsulfon. Bevorzugte Lösungsmittel sind Acetonitril, Äthylenchlorid und Chloroform.

Die Umsetzung kann bei Raumtemperatur oder höheren bzw. tieferen Temperaturen, vorzugsweise bei 0-100 °C, durchgeführt werden. Die Reaktionsteilnehmer werden im allgemeinen in annähernd äquimolarer Menge in die Reaktion eingesetzt, der Heterocyclus wird jedoch häufig in geringem Überschuss angewendet, um einen möglichst quantitativen Umsatz der Zuckerkomponente zu erreichen. Der Katalysator wird bevorzugt im molaren Überschuss (1,2-2,4 Äquivalente) eingesetzt. Das Silylierungsmittel wird, falls es für die *in situ* Bildung des Katalysators erforderlich ist, in entsprechendem Überschuß eingesetzt. Für die *in situ* Silylierung der Basen ist ein Überschuß an Silylierungsmittel von 0,05-0,5 Äquivalenten vorteilhaft.

Für die *in situ* Silylierung der heterocyclischen Basen muß für jede Hydroxy-, Mercapto- oder Aminogruppe in diesen Basen mindestens ein Äquivalent Trimethylchlorsilan (TCS) bzw. vorzugsweise ein Gemisch von 0,66 Äquivalenten Hexamethyldisilazan (HMDS) und 0,33 Äquivalenten TCS eingesetzt werden, welches zur Entstehung von 0,33 Äquivalenten $NH_4Cl$ führt.

Um die *in situ* Bildung der Trimethylsilylester der Perfluoralkansulfosäuren, der Perchlorsäure und der Borfluorwasserstoffsäure aus den entsprechenden Säuren oder Salzen zu bewerkstelligen, müssen den freien Säuren oder ihren Salzen äquivalente Mengen an Trimethylchlorsilan (TCS) bzw. Trimethylbromsilan (TBS) zugesetzt werden.

Die Katalysatoren in diesem neuen Verfahren sind identisch mit den früher verwendeten Katalysatoren- nur daß sie z.B. im Falle der Verwendung freier Perfluoralkansulfosäuren oder ihrer Salze bzw. der Salze der Perchlorsäure erst *in situ* nach folgenden Formeln unter Bildung von HCl oder HBr bzw. Natrium-, Kalium- oder Ammoniumchlorid gebildet werden.

$$C_4F_9SO_3H + (CH_3)_3SiCl \longrightarrow (CH_3)_3SiSO_3C_4F_9 + HCl$$
$$KSO_3C_4F_9 + (CH_3)_3SiCl \longrightarrow (CH_3)_3SiSO_3C_4F_9 + KCl$$
$$NH_4ClO_4 + (CH_3)_3SiCl \longrightarrow (CH_3)_3SiClO_4 + NH_4Cl$$
$$NaBF_4 + (CH_3)_3SiCl \longrightarrow (CH_3)_3SiBF_4 + NaCl$$

Als freie Säuren kommen infrage Wasserfreie Perfluoralkansulfonsäuren, insbesondere Trifluormethansulfonsäure und Perfluorbutansulfonsäure, Perchlorsäure sowie Borfluorwasserstoffsäure und ihre Salze, wie z.B. die Ammonium-, Natrium- und Kaliumsalze.

Die Ausbeuten dieser neuen, einstufigen Nucleosidsynthese, bei der sich überwiegend bzw. ausschließlich die β-Anomeren der Nucleoside bilden, liegen höher als bei den bisherigen Verfahren, wenn man die Verluste bei der früher üblichen getrennten Silylierung der organischen Base in Betracht zieht.

Zur Herstellung der Verbindungen mit freien Hydroxygruppen lassen sich die Schutzgruppen in üblicher Weise, z.B. durch alkoholische Lösungen von Ammoniak oder Alkoholaten, durch wäßriges oder alkoholisches Alkali sowie im Falle der Benzyläther durch Reduktion oder Hydrierung entfernen.

Die nachfolgenden Beispiele dienen der weiteren Erläuterung des erfindungsgemäßen Verfahrens.

Beispiel 1

0,56 g (5 mmol) Uracil, 2,52 g (5 mmol) 1-O-Acetyl-2,3,5-tri-O-benzoyl-β-D-ribofuranose sowie 4,06 g (12 mmol) Kaliumsalz der Perfluorobutansulfonsäure wurden in 70 ml abs. Acetonitril suspendiert, unter Rühren und Argon, 0,74 ml (3,5 mmol) Hexamethyldisilazan (HMDS) und 1,89 ml (15 mmol) Trimethylchlorsilan (TCS) zugefügt und 14 h am Rückfluß gekocht. Nach Verdünnen mit Methylenchlorid wurde mit ges. $NaHCO_3$-Lösung ausgeschüttelt und die Methylenchloridphase mit Wasser gewaschen. Nach Trocknen ($Na_2SO_4$), Abdampfen kristallisierte der braune Schaum (3,19 g) aus 95 % Athanol und ergab in mehreren Portionen 1,87 g reines Uridin-2',3',5'-tri-O-benzoat. Nach Chromatographie der Mutterlauge an Silikagel und Elution mit Toluol-Essigester (8 : 2) wurden weitere 0,45 g, insgesamt 2,32 g = 83,5 % reines Uridin-2',3',5'-tri-O-benzoat erhalten.

Beispiel 2

1,12 g (10 mmol) Uracil, 5,04 g (10 mmol) 1-O-Acetyl-2,3,5-tri-O-benzoyl-β-D-ribofuranose und 1,8 g (12 mmol) Trifluormethansulfonsäure wurden in 100 ml abs. Acetonitril mit 1,8 g = 2,3 ml (12 mmol) Hexamethyldisilazan (HMDS) und 1,3 g (12 mmol) Trimethylchlorsilan (TCS) versetzt, 4 h bei 24 °C gerührt und anschließend 1 1/2 h gekocht. Nach Aufarbeitung wie unter Beispiel 1 beschrieben wurden 4,50 g = 81 % kristallines Uridin-2',3',5'-tribenzoat erhalten.

Beispiel 3

0,56 g (5 mmol) Uracil, 2,52 g (5 mmol) 1-O-Acetyl-2,3,5-tri-O-benzoyl-β-D-ribofuranose wurden in 75 ml abs. Acetonitril mit 0,65 g (4 mmol) HMDS, 0,43 g (4 mmol) TCS und 1,56 g (6 mmol) $SnCl_4$ 2 h bei 24 °C gerührt. Aufarbeitung wie in Beispiel 1 lieferte 83,1 % kristallines Uridin-tribenzoat.

Beispiel 4

0,56 g (5 mmol) Uracil wurde in 70 ml abs. Acetonitril mit 1,86 g (3,5 mmol) HMDS und 2,55 g (15,5 mmol) TCS sowie 1,69 g (12 mmol) $NaClO_4 \cdot H_2O$ 30 min bei 24 °C gerührt, 2,52 g (5 mmol) 1-O-Acetyl-2,3,5-tri-O-benzoyl-β-D-ribofuranose zugesetzt und 20 h gekocht. Nach Aufarbeitung wie in Beispiel 1 wurden 58 % kristallines Uridin-tribenzoat erhalten.

Beispiel 5

0,56 g (5 mmol) Uracil, 2,52 g (5 mmol) 1-O-Acetyl-2,3,5-tri-O-benzoyl-β-D-ribofuranose wurden in 70 ml abs. Acetonitril mit 1,41 g (12 mmol) $NH_4ClO_4$, 0,57 g (3,5 mmol) HMDS und 1,68 g (15,5 mmol) TCS 20 h gekocht, Aufarbeitung wie in Beispiel 1 ergab 40 % kristallines Uridin-tribenzoat.

Beispiel 6

0,56 g (5 mmol) Uracil, 2,52 g (5 mmol) 1-O-Acetyl-2,3,5-tri-O-benzoyl-β-D-ribofuranose wurden in 70 ml abs. Acetonitril mit 1,32 g (12 mmol) $NaBF_4$, 0,57 g (3,5 mmol) HMDS und 1,68 g (15,5 mmol) TCS 2 h gekocht. Aufarbeitung wie in Beispiel 1 ergab 43 % kristallines Uridin-tribenzoat.

Beispiel 7

0,64 g (5 mmol) 2-Thiouracil, 2,52 g (5 mmol) 1-O-Acetyl-2,3,5-tri-O-benzoyl-β-D-ribofuranose, 4,06 g (12 mmol) Kaliumsalz der Perfluorbutansulfonsäure wurden in 70 ml abs. Acetonitril mit 0,57 g (3,5 mmol) HMDS sowie 1,68 g (15,5 mmol) TCS 17 h gekocht und wie in Beispiel 1 beschrieben aufgearbeitet. Dabei wurden 1,37 g (47,74 %) kristallines 2-Thiouridin-tribenzoat vom Schmelzpunkt 105-106 °C erhalten.

Beispiel 8

0,64 g (5 mmol) 2-Thiouracil, 2,52 g (5 mmol) 1-O-Acetyl-2,3,5-tri-O-benzoyl-β-D-ribofuranose wurden in 75 ml abs. Acetonitril mit 0,65 g (4 mmol) HMDS, 0,43 g (4 mmol) TCS und 1,56 g (6 mmol) $SnCl_4$ versetzt und nach 7 h/24 °C wie in Beispiel 1 beschrieben aufgearbeitet, wobei 64 % kristallines 2-Thiouridin-tribenzoat erhalten wurden.

Beispiel 9

0,63 g (5 mmol) 5-Methoxyuracil, 2,52 g (5 mmol) 1-O-Acetyl-2,3,5-tri-O-benzoyl-β-D-ribofuranose und 4,06 g (12 mmol) Kaliumsalz der Perfluorbutansulfonsäure wurden in 70 ml abs. Acetonitril mit 0,57 g (3,5 mmol) HMDS, 1,63 g (15 mmol) TCS 20 h gekocht und wie in Beispiel 1 aufgearbeitet. Kristallisation des Rohproduktes aus Essigester-Hexan ergab 2,09 g (71,4 %) kristallines 5-Methoxyuridin-2',3',5'-tri-O-benzoat.

Beispiel 10

0,55 g (5 mmol) Cytosin, 2,52 g (5 mmol) 1-O-Acetyl-2,3,5-tri-O-benzoyl-β-D-ribofuranose und 4,06 g (12 mmol) Kaliumnonaflat wurden in 70 ml abs. Acetonitril mit 0,57 g (3,5 mmol) HMDS, 1,68 g (15,5 mmol) TCS 27 h gekocht, wie üblich aufgearbeitet und mit Toluol-Essigester (3 : 2) an Silikagel chromatographiert, wobei 1,55 g (56 %) amorphes Cytidin-2',3',5'-tribenzoat erhalten wurden.

Beispiel 11

0,21 g (1,5 mmol) 2-Thio-5-methyluracil, 8,73 g Peracetylcellopentacosanose, 1,22 g Kaliumnonaflat wurden in 70 ml abs. Acetonitril mit 0,17 g (1,05 mmol) HMDS, 0,51 g (4,65 mmol) TCS 13 h gekocht, wie üblich aufgearbeitet (Emulsionen). Den Rückstand verseifte man mit 200 ml methanolischem $NH_3$ 3 Tage/24 °C, dampfte ab, extrahierte mit Äther und anschließend kochte man dreimal mit Methanol-$H_2O$ (1 : 1) aus und wusch mit Methanol. Der leicht graue Rückstand zeigte bei der Stickstoffbestimmung einen Stickstoffgehalt von 1,25 %.

Beispiel 12

0,64 g (5 mmol) 2-Thiouracil, 1,95 g (5 mmol) β-Pentaacetylglucose in 75 ml abs. Acetonitril wurden 2 h mit 0,65 g (4 mmol) HMDS, 0,43 g (4 mmol) TCS sowie 1,56 g (6 mmol) $SnCl_4$ gekocht, wie in Beispiel 1 aufgearbeitet und mit Toluol-Essigester (7 : 3) an Silikagel chromatographiert wobei 57 % 1-(2,3,4,6-Tetraacetyl-β-D-glucopyranosyl)-2-thiouracil erhalten wurden.

Beispiel 13

0,47 g (5 mmol) 4-Pyridon, 2,52 g (5 mmol) 1-O-Acetyl-2,3,5-tri-O-benzoyl-β-D-ribofuranose und 4,06 g (12 mmol) Kaliumnonaflat wurden mit 0,29 g (1,75 mmol) HMDS und 1,49 g (13,75 mmol) TCS in 70 ml abs. Acetonitril 12 h gekocht. Die übliche Aufarbeitung (Beispiel 1) ergab nach Chromatographie an Silikagel mit Essigester-Methanol (95 : 5) 65 % amorphes 1-(2',3',5'-Tri-O-benzoyl-β-D-ribofuranosyl)-4-pyridon.

Beispiel 14

1,19 g (5 mmol) $N^6$-Benzoyl-adenin, 2,52 g (5 mmol) 1-O-Acetyl-2,3,5-tri-O-benzoyl-β-D-ribofuranose und 4,06 g (12 mmol) Kaliumnonaflat wurden mit 0,57 g (3,5 mmol) HMDS und 1,68 g (15,5 mmol) TCS in 70 ml abs. Acetonitril 21 h gekocht und wie in Beispiel 1 aufgearbeitet. Das Rohprodukt (3,9 g) wurde in 100 ml methanolischen $NH_3$ 4 Tage bei 24 °C stehengelassen, abgedampft und der Rückstand nach Extraktion mit Äther aus wenig Wasser umkristallisiert, wobei 0,84 g (63 %) reines, kristallines Adenosin erhalten wurden.

Beispiel 15

0,56 g (5 mmol) Uracil wurden in 50 ml abs. Äthylenchlorid mit 0,65 g (4 mmol) HMDS, 0,43 g (4 mmol) TCS und 1,56 g (0,71 ml = 6 mmol) $SnCl_4$ 1 h gerührt, dann 2,52 g (5 mmol) 1-O-Acetyl-2,3,5-tri-O-benzoyl-β-D-ribofuranose zugefügt und weitere 2 h gerührt. Nach üblicher Aufarbeitung wurden 73 % kirstallines Uridin-2',3',5'-tri-O-benzoat erhalten.

Beispiel 16

0,65 g (5 mmol) 5-Fluoruracil, rührte man in 50 ml abs. Äthylenchlorid 1 h mit 0,65 g (4 mmol) HMDS, 0,43 g (4 mmol) TCS und 1,56 g (6 mmol) $SnCl_4$ und fügte dann 0,65 g (5 mmol) 2-Acetoxy-tetrahydrofuran oder 0,51 g (5 mmol) 2-Methoxytetrahydrofuran zu und rührte 2 bzw. 4 h bei 24 °C, arbeitete auf wie unter Beispiel 1 beschrieben und erhielt $N_1$-(2-Tetrahydrofuryl)-5-fluoruracil, Schmpt. 165-167 °C, in 87 % Ausbeute.

**Ansprüche**

1. Verfahren zur Herstellung von Nucleosiden durch Silylierung der entsprechenden Nucleosidbasen und Umsetzung mit einem 1-O-Acyl-, 1-O-Alkyl- oder 1-Halogen-Derivat eines geschützten Mono- oder Oligosaccharids in Gegenwart eines Katalysators, dadurch gekennzeichnet, daß man die Reaktion in einem Schritt durchführt.

2. Verfahren nach Anspruch 1, dadurch gekennzeichnet, daß man als Katalysator Friedel-Crafts-Katalysatoren insbesondere Trialkylsilylester, vorzugsweise Trimethylsilylester von Mineralsäuren oder starken organischen Säuren, die sich im Verlauf der Reaktion aus den freien Säuren oder ihren Salzen, insbesondere den Alkali- oder Ammoniumsalzen, mit Hilfe des im Überschuß zugesetzten Silylierungsmittels, insbesondere Trimethylchlorsilan, bilden, verwendet.

3. Verfahren nach Anspruch 1 und 2, dadurch gekennzeichnet, daß man als Katalysator die Friedel-Crafts-Katalysatoren $SnCl_4$, $TiCl_4$, $ZnCl_2$, $BF_3$-Ätherat, $AlCl_3$, $SbCl_5$ verwendet.

4. Verfahren nach Anspruch 1 und 2, dadurch gekennzeichnet, daß man als Katalysator die sich im Verlauf der Reaktion aus den entsprechenden freien Säuren oder ihren Salzen, insbesondere den Alkali- oder Ammoniumsalzen, mit Hilfe des im Überschuß zugesetzten Trimethylchlorsilans bildenden $(CH_3)_3SiBF_4$, $(CH_3)_3SiClO_4$, $(CH_3)_3SiSO_3C_nF_{2n+1}$, wobei $n = 1-10$ bedeutet, verwendet.

5. Verfahren nach Anspruch 1 bis 4, dadurch gekennzeichnet, daß man eine Nucleosidbase der allgemeinen Formel

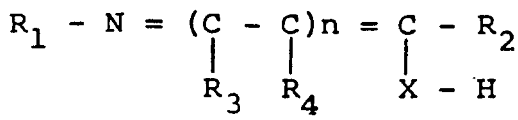

(Ia)

oder

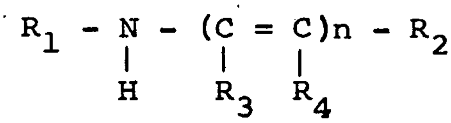

(Ib)

worin n die Zahl 0 oder 1 und X die Atome O oder S und $R_1$ und $R_2$ jeder für sich einen beliebigen gesättigten oder ungesättigten, gegebenenfalls substituierten organischen Rest oder gemeinsam einen zweiwertigen organischen Rest, der ein oder zwei Stickstoffatome enthalten kann, und $R_3$ und $R_4$ jeder für sich Wasserstoff, einen Alkyl-, Alkoxycarbonyl-, Alkylaminocarbonylrest oder gemeinsam die zweiwertigen Reste

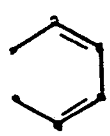 , 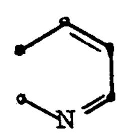 , 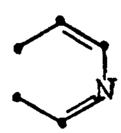 , 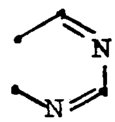 , 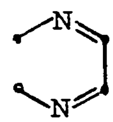 , 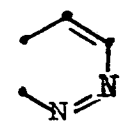 ,

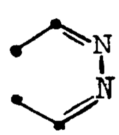 , 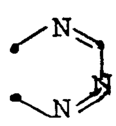 , 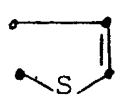 , 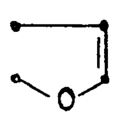 , 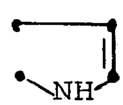 , 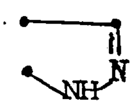

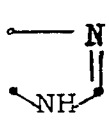 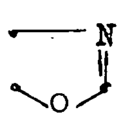 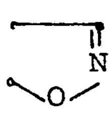 oder 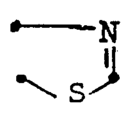

die in üblicher Weise substituiert sein können, verwendet.

6. Verfahren nach Anspruch 1 bis 5, dadurch gekennzeichnet, daß man eine Nucleosidbase der allgemeinen Formel

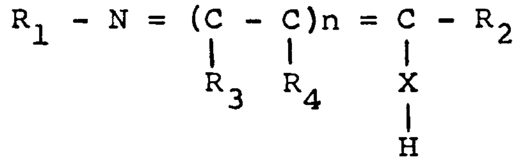

(Ia)

worin n gleich 1 ist, und $R_3$, $R_4$ und X die gleiche Bedeutung wie in Anspruch 5 haben, und $R_1$ und $R_2$ gemeinsam

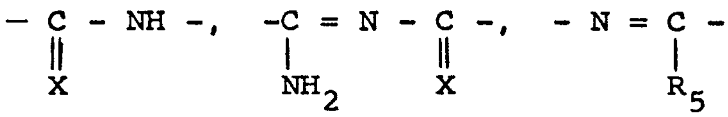

oder

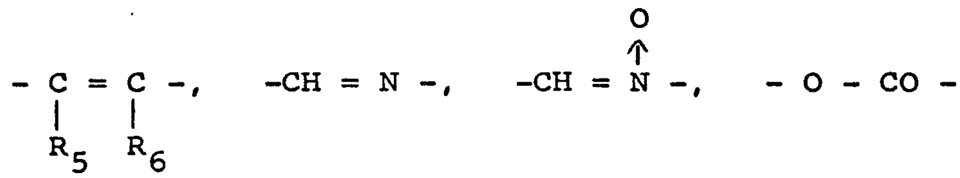

mit X in der in Anspruch 5 angegebenen Bedeutung und mit $R_5$ und $R_6$ in der Bedeutung eines Wasserstoffatoms, eines Alkyl-, Alkoxycarbonyl- oder Alkylaminocarbonylrestes, verwendet.

7. Verfahren nach Anspruch 1 bis 5, dadurch gekennzeichnet, daß man eine Nucleosidbase der allgemeinen Formel

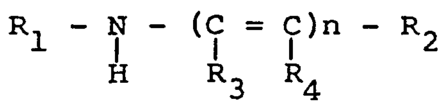

(Ib).

worin n gleich 0 ist und $R_3$ und $R_4$ das gleiche wie in Anspruch 5 und $R_1$ und $R_2$ gemeinsam

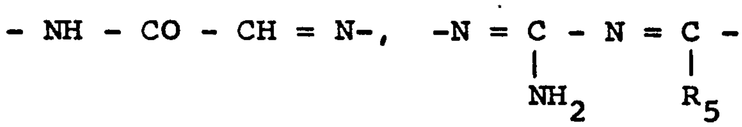

oder

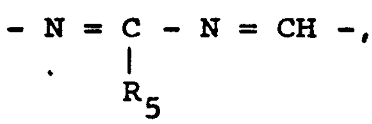

worin $R_5$ die in Anspruch 6 angegebene Bedeutung hat, bedeuten.

**Claims**

1. Process for the manufacture of nucleosides by silylation of the corresponding nucleoside bases and reacting with a 1-O-acyl, 1-O-alkyl or 1-halogen derivative of a protected mono- or oligo-saccharide in the presence of a catalyst, characterised in that the reaction are carried out in one step.

2. Process according to claim 1, characterised in that, as catalyst, there are used Friedel-Crafts catalysts, especially trialkylsilyl esters, preferably trimethylsilyl ester, of mineral acids or of strong organic acids, which trialkylsilyl esters are produced in the course of the reaction from the free acids or the salts thereof, especially from the alkali metal or ammonium salts, with the aid of a silylating agent which is added in excess, especially trimethylchlorosilane.

3. Process according to claims 1 and 2, characterised in that, as catalyst, there are used the Friedel-Crafts catalysts $SnCl_4$, $TiCl_4$, $ZnCl_2$, $BF_3$ etherate, $AlCl_3$, or $SbCl_5$.

4. Process according to claims 1 and 2, characterised in that, as catalyst, there are used $(CH_3)_3SiBF_4$, $(CH_3)_3SiClO_4$ and $(CH_3)_3SiSO_3C_nF_{2n+1}$ in which n is 1 to 10, which are produced in the course of the reaction from the corresponding free acids or the salts thereof, especially from the alkali metal or ammonium salts, with the aid of the trimethylchlorosilane which is added in excess.

5. Process according to claims 1 to 4, characterised in that, there is used a nucleoside base of the general formula

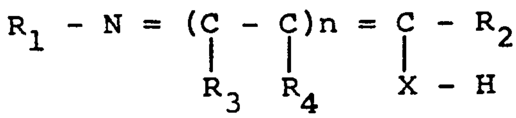 (Ia)

or

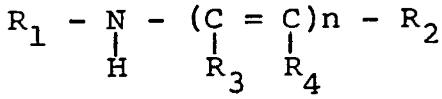 (Ib)

in which n is the number 0 or 1, X represents the atoms O or S, $R_1$ and $R_2$ each represents any desired saturated or unsaturated, optionally substituted, organic radical or together represent a bivalent organic radical that may contain one or two nitrogen atoms, and $R_3$ and $R_4$ each represents hydrogen, or an alkyl alkoxycarbonyl or alkylaminocarbonyl radical, or together represent the bivalent radicals

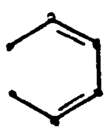, 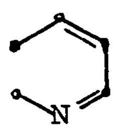, 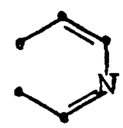, 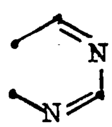, 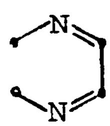, 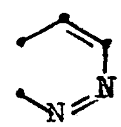,

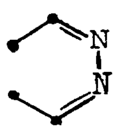, 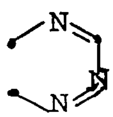, 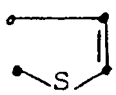, 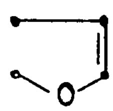, 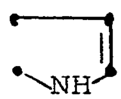, 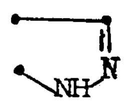

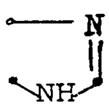 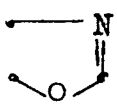 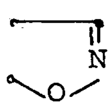 or 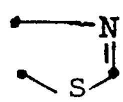

which may be substituted in usual manner.

6. Process according to claims 1 to 5, characterised in that there is used a nucleoside base of the general formula

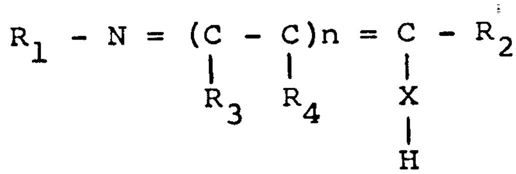 (Ia)

in which n is 1, $R_3$, $R_4$ and X have the same meaning as in claim 5, and $R_1$ and $R_2$ together represent

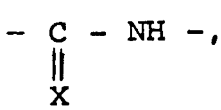, 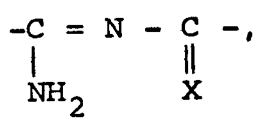, 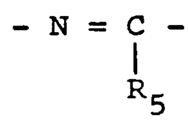 or

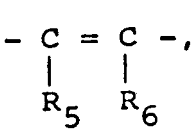, $-CH=N-$, 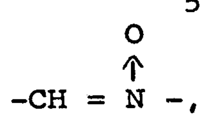, $-O-CO-$

in which X has the meaning given in claim 5, and $R_5$ and $R_6$ represent a hydrogen atom, or an alkyl, alkoxycarbonyl or alkylaminocarbonyl radical.

7. Process according to claims 1 to 5, characterised in that there is used a nucleoside base of the general formula

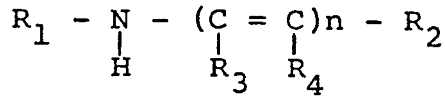
(Ib)

in which n is 0, $R_3$ and $R_4$ are the same as in claim 5, and $R_1$ and $R_2$ together represent

$-NH-CO-CH=N-$, 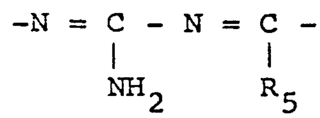 or

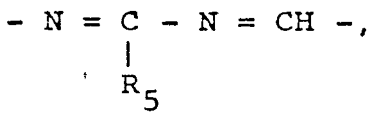,

in which $R_5$ has the meaning given in claim 6.

**Revendications**

1. Procédé de préparation de nucléosides par silylation des bases correspondantes des nucléosides et réaction avec un dérivé 1-O-acylé, 1-O-alkylé ou 1-halogéné d'un monosaccharide ou d'un oligosaccharide protégé en présence d'un catalyseur, procédé caractérisé en ce que l'on effectue la réaction en un seul stade.

2. Procédé selon la revendication 1, caractérisé en ce que l'on utilise des catalyseurs de Friedel et Crafts, en particulier des esters trialkylsilyliques, de préférence triméthylsilyliques, d'acides minéraux ou d'acides organiques forts, qui se forment au cours de la réaction à partir des acides libres ou de leurs sels, notamment de leurs sels de métaux alcalins ou d'ammonium, avec l'agent de silylation ajouté en excès, en particulier le triméthylchloro-silane.

3. Procédé selon la revendication 1 ou 2, caractérisé en ce que l'on utilise les catalyseurs $SnCl_4$, $TiCl_4$, $ZnCl_2$, l'éthérate de $BF_3$, $AlCl_3$ ou $SbCl_5$.

4. Procédé selon la revendication 1 ou 2, caractérisé en ce que l'on utilise, comme catalyseurs qui se forment au cours de la réaction à partir des acides libres correspondants ou de leurs sels, en particulier des sels alcalins ou d'ammonium, avec le triméthylchlorosilane ajouté en excès, $(CH_3)_3SiBF_4$, $(CH_3)_3SiClO_4$ ou $(CH_3)_3SiSO_3 \cdot C_nF_{2n+1}$ (avec n = 1 à 10).

5. Procédé selon l'une quelconque des revendications 1 à 4, caractérisé en ce que l'on utilise une base de nucléoside de formule générale

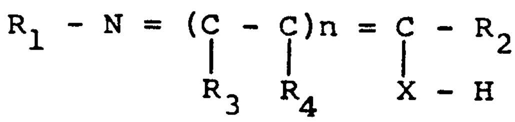
(Ia)

ou

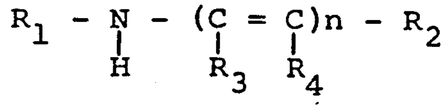
(Ib)

formules dans lesquelles n est le nombre 0 ou 1 et X représente l'atome d'oxygène ou de soufre, $R_1$ et $R_2$ désignent chacun un radical organique quelconque saturé ou insaturé, éventuellement substitué, ou bien représentent ensemble un radical organique divalent pouvant comporter un ou deux atomes d'azote, et $R_3$ et $R_4$ désignent chacun l'hydrogène ou un groupe alkyle, alcoxycarbonyle ou alkylaminocarbonyle, ou bien représentent ensemble le radical divalent

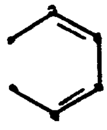, 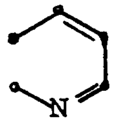, 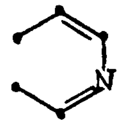, 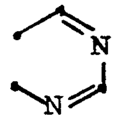, 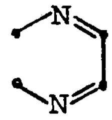, 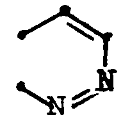

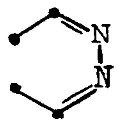 , 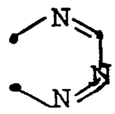 , 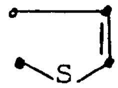 , 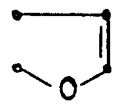 , 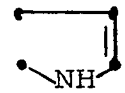 , 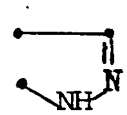

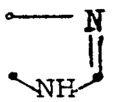 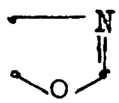 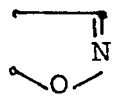 ou 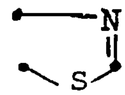

pouvant avoir les substituants habituels.

6. Procédé selon l'une quelconque des revendications 1 à 5, caractérisé en ce que l'on utilise une base de nucléoside de formule générale

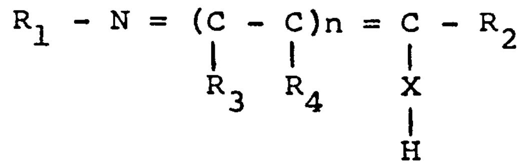 (Ia)

dans laquelle n = 1 et les symboles $R_3$, $R_4$ et X ont les significations données à la revendication 5, et $R_1$ et $R_2$ représentent ensemble

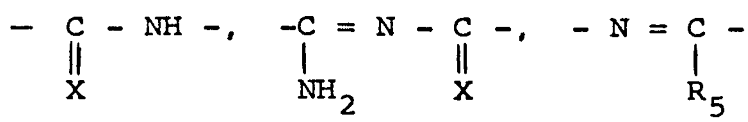 ou

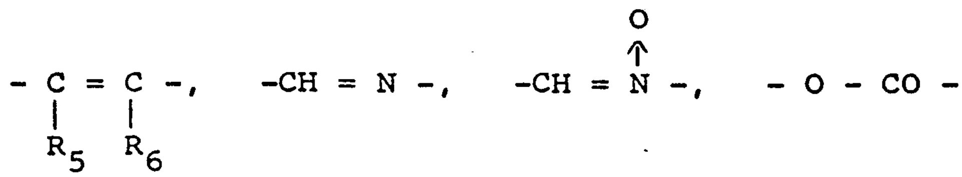

X ayant la même signification que dans la revendication 5 et $R_5$ et $R_6$ ayant chacun un atome d'hydrogène ou un groupe alkyle, alcoxycarbonyle ou alkylaminocarbonyle.

7. Procédé selon l'une quelconque des revendications 1 à 5, caractérisé en ce que l'on utilise une base de nucléoside de formule générale

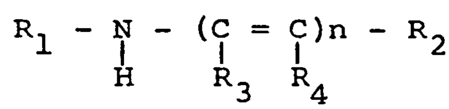 (Ib)

dans laquelle n = 0 et $R_3$ et $R_4$ ont les mêmes significations que dans la revendication 5, et $R_1$ et $R_2$ représentent ensemble

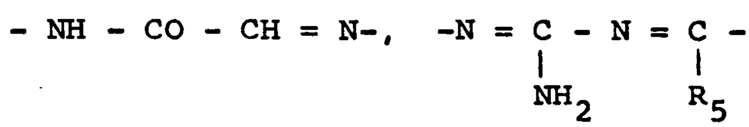 ou

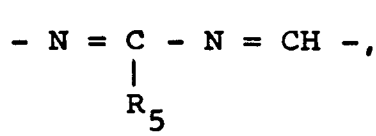

$R_5$ ayant la signification donnée à la revendication 6.